(19) **Europäisches Patentamt European Patent Office Office européen des brevets**

(11) **EP 3 019 008 B1**

(12) **EUROPEAN PATENT SPECIFICATION**

(45) Date of publication and mention
of the grant of the patent:
**02.03.2022 Bulletin 2022/09**

(21) Application number: **14739710.3**

(22) Date of filing: **02.07.2014**

(51) International Patent Classification (IPC):
*A01N 31/02* (2006.01)    *A61K 8/34* (2006.01)
*A61Q 17/00* (2006.01)    *A61K 31/045* (2006.01)
*A61K 31/047* (2006.01)    *A01P 1/00* (2006.01)

(52) Cooperative Patent Classification (CPC):
(C-Sets available)
**A61Q 17/005; A01N 31/02; A61K 8/34;
A61K 8/342; A61K 8/345; A61K 31/045;
A61P 17/00; A61P 31/02; A61P 31/12;**
A61K 2800/30                    (Cont.)

(86) International application number:
**PCT/EP2014/064071**

(87) International publication number:
**WO 2015/000961 (08.01.2015 Gazette 2015/01)**

(54) **USE OF AN ISOPROPANOL-CONTAINING SOLUTION TO COMBAT CALCIVIRIDAE**

VERWENDUNG EINER ISOPROPANOLHALTIGEN LÖSUNG ZUR BEKÄMPFUNG VON
CALCIVIRIDAE

UTILISATION D' UNE SOLUTION CONTENANT DE L'ISOPROPANOL POUR COMBATTRE
CALCIVIRIDAE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.07.2013 DE 102013213252**

(43) Date of publication of application:
**18.05.2016 Bulletin 2016/20**

(73) Proprietor: **Schülke & Mayr GmbH
22851 Norderstedt (DE)**

(72) Inventors:
• **DA SILVA NOLASCO, Angelo**
**22111 Hamburg (DE)**
• **GORONCY-BERMES, Peter**
**22145 Hamburg (DE)**
• **OLTMANNS, Peter**
**27499 Hamburg (DE)**
• **STEINHAUER, Katrin**
**22459 Hamburg (DE)**
• **KOLDITZ, Petra**
**22145 Hamburg (DE)**

(74) Representative: **LKGLOBAL
Lorenz & Kopf PartG mbB Patentanwälte
Brienner Straße 11
80333 München (DE)**

(56) References cited:
**EP-A1- 0 176 720        EP-A1- 2 135 507
EP-A1- 2 462 806        WO-A1-03/018067
JP-A- 2011 173 815        US-A- 5 441 723**

• **ALLEGRANZI B ET AL: "Role of hand hygiene in
healthcare-associated infection prevention",
JOURNAL OF HOSPITAL INFECTION,
ACADEMIC PRESS, LONDON, GB, vol. 73, no. 4,
31 August 2009 (2009-08-31), pages 305-315,
XP026797133, ISSN: 0195-6701 [retrieved on
2009-08-31]**

(52) Cooperative Patent Classification (CPC): (Cont.)

C-Sets
**A01N 31/02, A01N 25/04**

**Description**

The present invention relates to an isopropanol-containing composition and its use in a method of combating Caliciviridae (caliciviruses) on the skin. In addition, the invention relates to the use of this composition to combat Caliciviridae on non-living surfaces.

The combating of viruses plays a crucial role in a large number of technical fields. Caliciviridae, and in particular noroviruses, have become the focus of interest in recent years, in particular in disinfection in hospitals. Disinfection of the hands and of surfaces are in this case essential methods of breaking the infection chain.

EP 0 547 727 A1 describes compositions which can be used as a skin antiseptic and a hand disinfection agent. Effectiveness against viruses is not described. WO 98/44792 A1 discloses the effectiveness of alcohols (in particular ethanol and n-propanol) when mixed with salts at a low temperature against viruses.

EP 2 135 507 A1 discloses a virucidal disinfectant which comprises (a) 40 to 90% by weight ethanol, isopropanol or a mixture thereof, (b) 0.1 to 2% by weight of lactic acid, (c) 0.01 to 2% by weight of citric acid and (d) 0.001 to 0.1% by weight of a zinc containing compound which releases zinc in solution.

EP 1 685 854 A1 discloses a virucidal composition comprising (a) 0.2 to 1.5% by weight of one or several phosphorous containing acid, (b) 30 to 80% by weight, of ethanol, propanol-1, propanol-2 or of a mixture of two or three of them and (c) one or several polyalkylenglycol.

JP 200035506 A disclose a germicidal composition mild to the human body which comprises (A) 30 to 90 Vol.% alcohols such as ethanol, isopropanol or mixture thereof, (B) 0.01 to 5 Vol.% moisturizing agent such as glycerol, triglycerol, ethyleneglycol, a polyethylene glycol sorbitol ot a POE methyl glycoside and (C) water in the balance.

By way of example, disinfecting agents based upon ethanol and acting as a virucide are recommended for the hygiene and disinfection of the hands. A virucidal activity has been shown for the product desderman® pure of Schülke & Mayr GmbH (78% by weight of ethanol 96%, 0.1% by weight of biphenyl-2-ol). There are nevertheless reservations concerning disinfecting agents for hygienic and surgical hand disinfection which contain large quantities of ethanol, since agents of this type have a strong degreasing effect.

The product desmanol® industrial pure of Schülke & Mayr GmbH, on the other hand, contains 32% by weight of propanol-1 (n-propanol) and 21% by weight of propanol-2 (isopropanol). The product is used undiluted for hand disinfection and is effective against lipophilic viruses. Agents with a content $\geq$ 3% n-propanol, however, must be classified as R41 ("risk of serious damage to eyes"), together with the symbol "irritant"; from the 1st of June 2015 preparations containing $\geq$ 3% n-propanol must be classified as H318 (GHS classification "causes serious eye damage") and a "corrosive" symbol.

In order to combat viruses in the disinfection of surfaces, the Robert Koch Institute (the German Federal Institute for Infectious Diseases and Non-Communicable Diseases) recommends disinfection agents with a content of per-compounds or aldehydes as active substances (Epidemiological Bulletin 4/2009). In addition, in accordance with *Wallhäußers Praxis der Sterilisation, Desinfektion, Antiseptik und Konservierung* [Wall-häusser's Practice of Sterilization, Disinfection, Antiseptics and Conservation] (Kramer & Assadian, Publ. Thieme Verlag 2008) only substances with an oxidizing action, such as aldehydes, halogens (in particular, active chlorine) and compounds releasing active oxygen (such as peracids, perborates and hydrogen peroxides), are effective against non-enveloped viruses.

The human norovirus causes more than 80% of all types of infectious gastroenteritis. It has been shown in various tests that the effectiveness of isopropanol against noroviruses is limited. In addition, for combating noroviruses on non-living surfaces, Magulski et al. (BMC-Infectious Diseases 2009, 9:107) therefore recommend products on the basis of peracetic acid, glutaraldehyde, ethanol or n-propanol. In the tests by Magulski *et al.*, the murine norovirus was used as a surrogate for the human norovirus. In the highest concentration tested of 60% by volume the effectiveness of isopropanol lay three decadic levels below the effectiveness of ethanol and n-propanol. These results are also confirmed by Eggers (International Conference on Prevention and Infection Control, Schülke Satellite Symposia, Geneva 2011).

The subject-matter of the present invention is a disinfectant which is active against Caliciviridae to be used for skin, hand and surface disinfection. The disinfection agent is effective against noroviruses, and more particularly against the murine norovirus. Furthermore, the disinfectant is easy to formulate and has the least possible degreasing effect when used for hygienic and surgical hand disinfection.

That is the reason why, according to a first embodiment, the invention relates to a composition which contains:

a) - At least 60% by weight of isopropanol, and
b) - At least 0.2% by weight of one or more relipidizing agents.

The invention is based *inter alia* upon the fact that it has surprisingly been found that the effectiveness against Caliciviridae of the composition as hereinabove defined disinfection agents based upon isopropanol and relipidizing agents is similar to the effectiveness of disinfection agents based upon ethanol. This result was not predictable since it had previously been assumed that even in a high concentration isopropanol is not sufficient to destroy Caliciviridae.

Moreover, it has additionally been found that relipidizing agents as component b) do not inhibit the effect of isopropanol against Caliciviridae.

In this case the evidence of the effect upon the skin in a practical trial on test persons is decisive. This effect is surprising since, although previous experiments in the suspension test had proved a 4-log level reduction, previous experiments on the skin had shown the opposite. Hitherto, therefore, the effectiveness of isopropanol upon the murine norovirus (MNV) and the feline calicivirus (FCV) had been shown only in tests *in vitro* (suspension tests). The fact that conclusions on the effectiveness upon the skin cannot be drawn directly from the results of suspension tests, however, had already been shown by Eggers (*loc. cit.*).

In the hereinabove defined composition, the content of isopropanol preferably amounts at most to 95% by weight and preferably at most to 90% by weight, preferably from 65% to 85% by weight, in particular from 70% to 80 % by weight, for example from 72% to 78% by weight, such as approximately 75% by weight.

In the hereinabove defined composition, by a relipidizing agent, it is referred to "ein Rückfetter" in German language. The total content of b) relipidizing agents preferably amounts at most to 4.0% by weight, preferably from 0.5 to 2.5% by weight, in particular from 0.8 to 2.0% by weight.

Therefore, according to a particular embodiment, the composition as hereinbefore defined, contains:

> a) - At least 60% by weight and at most to 95% by weight of isopropanol, and
> b) - At least 0.2% by weight and at most 4.0% by weight of one or more relipidizing agents; it particularly contains:

>> a) - 60% by weight to 90% by weight of isopropanol, and
>> b) - 0.2% by weight to 2.5% by weight of one or more relipidizing agents; and more particularly contains:

>>> a) - At least 65% by weight and at most to 85% by weight of isopropanol, and
>>> b) - At least 0.5% by weight and at most 2.5% by weight of one or more relipidizing agents.

According to a very particular embodiment of the composition as hereinbefore defined, contains:

> a) - At least 70% by weight and at most to 80% by weight of isopropanol, and
> b) - At least 0.5% by weight and at most 2.5% by weight of one or more relipidizing agents.

By way of example, the relipidizing agents are selected from:

> (i) - (partial) esters of (poly)glycerol, for example glycerol (mono-, di-, tri-)[adipate, alkanoate ($C_8$, $C_{10}$ and $C_{18}$), isostearate], triglycerol diisostearate, polyglycerol-2 dipolyhydroxystearate, glycerol oleate, und glycerol cocoate,
> (ii) - lanolin and lecithin as well as polyethoxylated or acylated lanolin and lecithin derivatives,
> (iii) - polyols such as glycerol, erythritol, 1,2,6-hexanetriol, inositol, lactitol, maltitol, mannitol, methylpropanediol, phytantriol, polyglycerols, sorbitol and xylitol, wherein glycerol is particularly preferred,
> (iv) - fatty alcohols, *i.e.,* univalent, primary alcohols derived from unbranched, optionally mono- or polyunsaturated hydrocarbon residues with from 6 to 22 carbon atoms, such as lauryl alcohol, myristyl alcohol, cetyl alcohol and stearyl alcohol, and
> (v) - esters such as sorbitan laurate, isopropyl myristate, isopropyl palmitate and cetyl stearyl acetate.

According to another particular embodiment, the composition as hereinbefore defined, is characterized in that the component b) is glycerol and more particularly in that it comprises at most 2% by weight of glycerol, preferably at most 1.0% by weight and in particular, at most 0.5% by weight of glycerol.

According to another particular embodiment, the composition as hereinbefore defined, is characterized in that the component b) is a mixture of at least two or more fatty alcohols. According to this embodiment said mixture of two or more fatty alcohols is a mixture of cetyl alcohol and of stearyl alcohol or a mixture of cetyl alcohol, stearyl alcohol and myristyl alcohol.

According to another particular embodiment, the composition as hereinbefore defined, is characterized in that it further comprises at least another relipidizing agent different from glycerol. According to this embodiment said at least another relipidizing agent different from glycerol is a mixture of cetyl alcohol, stearyl alcohol and myristyl alcohol.

According to another particular embodiment the composition as hereinbefore defined further contains:
c) - From 0.05 to 2.0% by weight of at least one or several further ingredients.

Such ingredients are for example humectants such as sorbitol, and urea and/or dexpanthenol as a skin protection and a medicinal skin care agent or agents. In addition, glycerol ether can be contained in the disinfection agent, as disclosed in EP 0547 727 A, in particular 1-(2-ethylhexyl) glycerinether (Sensiva® SC50).

According to this embodiment, the composition is more particularly characterized in that said at least one several ingredients is chosen from sorbitol, urea, dexpanthenol, 1-(2-éthyl hexyl) glycerol ether, a combination of sorbitol and despanthenol, or a combination of sorbitol, dexpanthenol and 1-(2-éthyl hexyl) glycerol ether.

According to a more particular embodiment, the composition as hereinabove defined, is characterized in that it consists of, for 100% by weight:

a) - At least 60% by weight and at most to 95% by weight of isopropanol, and
b) - At least 0.2% by weight and at most 4.0% by weight of one or more relipidizing agents,
c) - From 0.05 to 2.0% by weight of at least one or several further ingredients.
d) - Water as the remainder.

According to another embodiment, the invention relates to the composition as hereinabove defined, for use in a method for treatment of the human or animal body by therapy, and more particularly for use as a skin disinfectant in a method for treatment of the human or animal body by therapy.

Said composition is effective against Caliciviridae (caliciviruses). These are mainly viruses of the genus norovirus, and in particular the human norovirus or the murine norovirus. Alternatively, the Caliciviridae are viruses of the genus vesivirus, and in particular the feline calicivirus.

Most preferably, the composition as hereinabove defined is for use in the treatment of the human skin, such as the hands,

According to the last embodiment, the invention relates to the use of a composition as hereinabove defined, as a disinfectant of non living surface, for example for area disinfection.

The disinfection agent used according to the invention thus provides the following advantages:

- good effectiveness against a multiplicity of micro-organisms, in particular against Caliciviridae, such as noroviruses or vesiviruses, at the same time as good compatibility with the skin (even with repeated applications),
- good effectiveness within a short exposure time, and
- effectiveness against Caliciviridae on living and non-living surfaces.

The advantages of the present invention are more particularly shown inthe following examples. Percentages refer to the weight, unless indicated otherwise.

## Examples

The following formulations were tested:

|  | Formulation A | Formulation B | Formulation C |
|---|---|---|---|
| Purified water, % | 23.30 | 23.50 | 24.00 |
| Isopropanol, % | 75.00 | 75.00 | 74.00 |
| Glycerol,% | - | - | 2·00 |
| Sorbitol 70%,% | 0.50 | 0.50 | - |
| 1-(2-ethylhexyl) glycerin ether, % | 0.20 |  |  |
| Dexpanthenol,% | 0.20 | 0.20 | - |
| Myristyl alcohol,% | 0.50 | 0.50 | - |
| Cetyl stearyl alcohol,% | 0.30 | 0.30 | - |
| Appearance | clear, colourless | colourless, clear | colourless, clear |

**Method A:Quantitative suspension test of virucidal effect according to EN 14476: Murine norovirus (low stressing)**

Storage conditions: in the original packaging at room temperature
Packaging: plastic bottle

**Methodology**:EN 14476:2005-04/A1:2006-10 and PrEN 14476:2011: Chemical disinfection agents and antiseptics - Quantitative suspension test of virucidal effect for chemical disinfection agents and antiseptics used in human medicine, Test methods and requirements (phase 2, stage 1)

For the demonstration of the virucidal effectiveness according to EN 14476, incubation of suspensions of 8 parts by volume of the disinfection agent (product test solution) with one part of stressing substance (0.3% in the testing of dirty conditions or 0.03% in the testing of clean conditions) and one part by volume of virus suspension is carried out with a titre of at least $10^7$ TC ID 50 / ml. In order to achieve the concentration of 97%, 0·1 ml of the virus, 0.2 ml of the 5-fold concentrated stressing substance and 9.7 ml of the product are mixed. After the termination of the exposure time, the effect of the disinfection agent is stopped by dilution (or gel filtration) and a series of dilutions in accordance with the progression 1:10/ 1:100 / 1:1,000 *etc.* is applied in order to determine the residual virus content. The titration is carried out in the microtitre system on 96-well plates. In addition, the following controls are carried out: virus control, cytotoxicity control, susceptibility control and reference inactivation with 0.7% formaldehyde. The calculation of the results is carried out in accordance with Spearman and Kärber and is calculated as the difference between the titre of the virus control and the titre of the product test solution whilst including a 95% confidence interval.

**Test conditions**

| | |
|---|---|
| Exposure time: | 30 sec, 60 sec, 120 sec |
| Stressing: | 0.3 g BSA/l (low stressing) |
| Test temperature: | 20°C ± 0.5°C |
| Concentration of the Test product (V/V): | 97% (V/V), 80% (V/V), 50% |
| Dilution agent: | water of standardized hardness |
| Test virus: | murine norovirus, strain S 99 Berlin |
| Cell line for propagation: | RAW cells ATCC TIB-71 |

Titration method:

Virus titration on cells as monolayers in 96-well microtitration plates. 0.5ml of product test solution is diluted with 4.5 ml of ice-cold DMEM + 2% FBS to a dilution of $10^{-8}$. 100 µl of each dilution was pipetted into 6 wells of the microtitration plate. Method of terminating the effect of the product and reducing the cytotoxicity:
Gel filtration with MicroSpin columns and dilution to $10^{-4}$ within 10 sec with ice-cold DMEM + 2% FBS

Susceptibility testing:
Comparative virus titrations were carried out on cells which had been treated with 0.1% product solution or PBS.

| | |
|---|---|
| Reference virus inactivation: | 0.7% formaldehyde solution. |
| Exposure times: | 5, 15, 30, 60 min |

**Calculation of the virucidal effectiveness**

The $TCID_{50}$ was calculated in accordance with the method of Spearman and Kärber.

$$m = x_k + d/2 - d \sum p_i$$

m = negative logarithm of the titre in relation to the test volume
$X_k$ = logarithm of the smallest dilution stage in which all the test objects react positively
d = logarithm of the dilution factor
$p_i$ = observed reaction rate

**Calculation of the standard deviation**

$$S_m = \sqrt{d^2 \sum [P_i \times (1 - P_i) \div (n - 1)]}$$

$S_m$ = standard deviation of the logarithmized titre
d = logarithm of the dilution factor
$p_i$ = observed reaction rate
n = number of tests per dilution

**Calculation of the reduction factor (RF)**

$$RF = \log(\text{virus control}) - \log(\text{test product})$$

$$K_{RF} = \sqrt{(2S_{m(\text{control})})^2 + (2S_{m(\text{residual titre})})^2}$$

$K_{RF}$ = 95% confidence interval of the reduction factor
$S_m$ = standard deviation of the logarithmized titre

A summary of the results of the quantitative suspension test in accordance with EN 14476 with the preferred formulation A and murine norovirus is given in Tables 1 and 2. The results of the controls are summarized in Table 3.

**Method B:: Modified test EN 1500 "Hygienic disinfection of the hands"**

The test is based upon EN 1500 (practical test method for the hygienic disinfection of the hands).
In the tests, the hands of test persons are contaminated with 3 ml of murine norovirus, namely both the hands of the test persons of the reference group and the hands of the test persons of the test group. A sample is then taken (1 min) before the disinfection. After that, the test persons of each group disinfect their hands with disinfection agent. The test persons of the reference group disinfect twice with 3 ml of a reference product in each case, the reference product being allowed to act for 30 sec and rubbed in after each disinfection. The test persons of the test group instead disinfect twice with 3 ml of the disinfection agent to be tested. After that, a sample is taken for the disinfection (1 min), both in the reference group and in the test group.
The test persons of the respective group are then swapped over in a second test.

**Example 1**

**Results of murine norovirus (method A)**

Table 1: Summary of the results of formulation A and murine norovirus

| Product concentration | Stressing | CD50 | $\log_{10}$-TCID$_{50}$/ 0.1 ml after ... sec murine norovirus | | | Titre reduction $\geq$ 4 $\log_{10}$ after... sec |
|---|---|---|---|---|---|---|
| | | | 30 | 60 | 120 | |
| 97% formulation A | 0.3 g BSA/l | $\leq$ 2.50 | $\leq$ 2.50 $\pm$ 0.00 | $\leq$ 2.50 $\pm$ 0.00 | $\leq$ 2.50 $\pm$ 0.00 | 30 |
| 80% formulation A | 0.3 g BSA/l | $\leq$ 2.50 | $\leq$ 2.50 $\pm$ 0.00 | $\leq$ 2.50 $\pm$ 0.00 | $\leq$ 2.50 $\pm$ 0.00 | 30 |
| 50% formulation A | 0.3 g BSA/l | $\leq$ 2.50 | 6.00 $\pm$ 0.42 | 5.50 $\pm$ 0.00 | 5.83 $\pm$ 0.42 | - |
| Virus control 1 ml virus | 0.3 g BSA/l | n.a. | n.c. | n.c. | 7.50 $\pm$ 0·47 | n.a. |

(continued)

| Product concentration | Stressing | CD50 | log$_{10}$-TCID$_{50}$/ 0.1 ml after ... sec murine norovirus | | | Titre reduction $\geq$ 4 log$_{10}$ after... sec |
|---|---|---|---|---|---|---|
| Virus control 0·1 ml virus | 0.3g BSA/I | n.a. | n.c. | n.c. | 6.33 $\pm$ 0.54 | n.a. |
| TCID$_{50}$ tissue culture infectious dose<br>CD$_{50}$ cytotoxic dose<br>n.a. not applicable<br>n.c. not carried out<br>BSA bovine serum albumin | | | | | | |

## Results of murine norovirus (method A)

Table 2: Summary of the results of formulation A and murine norovirus

| Product concentration | Stressing | CD50 | Virus control [log$_{10}$-TCID$_{50}$ / 0.1 ml] murine norovirus | Reduction factor [log$_{10}$-TCID$_{50}$ / 0·1 ml] | | |
|---|---|---|---|---|---|---|
| | | | | 30 | 60 | 120 |
| 97% formulation A | 0.3 g BSA/I | $\leq$ 2.50 | 6.33 $\pm$ 0.54 | $\geq$ 3.83 $\pm$ 0.54 | $\geq$ 3.83 $\pm$ 0.54 | $\geq$ 3.83 $\pm$ 0.54 |
| 80% formulation A | 0.3 g BSA/I | $\leq$ 2.50 | 7.50 $\pm$ 0.47 | $\geq$ 5.00 $\pm$ 0.47 | $\geq$ 5.00 $\pm$ 0.47 | $\geq$ 5.00 $\pm$ 0.47 |
| 50% formulation A | 0.3 g BSA/I | $\leq$ 2.50 | | 1.50 $\pm$ 0.63 | 2.00 $\pm$ 0.47 | 1.67 $\pm$ 0.63 |
| TCID$_{50}$ tissue culture infectious dose<br>CD$_{50}$ cytotoxic dose<br>BSA bovine serum albumin | | | | | | |

## Results of murine norovirus (method A)

Table 3: Virus controls and reference inactivation with murine norovirus

| Product concentration | Stressing | CD50 | log$_{10}$-TCID$_{50}$ / 0·1 ml after ... min murine norovirus | | | | |
|---|---|---|---|---|---|---|---|
| | | | 0 | 5 | 15 | 30 | 60 |
| Formaldehyde 0·7% (V/V) | PBS | $\leq$ 3.5 | n.c. | 6.33 $\pm$ 0.54 | 5.67 $\pm$ 0.56 | 5.17 $\pm$ 0.56 | 4.33 $\pm$ 0.33 |
| Virus control 20°C 1 ml virus | 0.3 g BSA/I | n.a. | 7.50 $\pm$ 0·47 | n.c. | n.c. | n.c. | n.c. |
| Virus control 20°C 0·1 ml virus | 0.3 g BSA/I | n.a. | 6.33 $\pm$ 0.54 | n.c. | n.c. | n.c. | n.c. |
| Susceptibility (PBS) | 0.3 g BSA/I | n.a. | n.c. | n.c. | n.c. | n.c. | 7.67 $\pm$ 0.62 |
| Susceptibility (0·1% formulation A) | 0.3 g BSA/I | n.a. | n.c. | n.c. | n.c. | n.c. | 7.67 0.33 |
| n.a. not applicable<br>n.c. not carried out | | | | | | | |

(continued)

| Product concentration | Stressing | CD50 | log$_{10}$-TCID$_{50}$ / 0·1 ml after ... min murine norovirus | | | | |
|---|---|---|---|---|---|---|---|
| | | | 0 | 5 | 15 | 30 | 60 |
| WSH water of standardized hardness<br>PBS phosphate-buffered salt solution<br>TCID$_{50}$ tissue culture infectious dose<br>CD$_{50}$ cytotoxic dose | | | | | | | |

### Verification of the method

| | |
|---|---|
| Cytotoxicity: | After gel filtration, the 97%, 80% and 50% products of formulation A with a dilution of 10$^{-2}$ (2.5 CD$_{50}$) displayed no cytotoxic effect. This consequently does not affect the necessary dilutions to prove a virucidal effect. |
| Virus control: | The virus titre for the murine norovirus amounted to 7.50 $\pm$ 0.47 TCID$_{50}$ / 0·1 ml and thus to 8.50 $\pm$ 0.47 TCID$_{50}$ / ml. The demonstrable titre reduction thus amounted to $\geq$ 4.00 lg$^{10}$. |
| Susceptibility: | The comparative virus titration on pre-treated and untreated cells displayed a difference of < 1.00 log$^{10}$ levels. |
| Reference inactivation | The reduction factor of 0.7% formaldehyde amounted to 2·33 log$^{10}$ after 30 min exposure time and to 3.17 log$^{10}$ after 60 min exposure time. |

### Results

After an exposure time of 30 sec with the 97% formulation A with a low stressing, no propagation of murine norovirus could be shown in cultures of RAW cells. It was already possible to demonstrate the necessary titre reduction of 4 log10 levels for an 80% application concentration.

### Example 2 (method B)

| | Formulation B | Formulation C | Ethanol 70% w/w |
|---|---|---|---|
| **log TCID50/ml** | **2 x 30 s** | **2 x 30 s** | **2 x 30 s** |
| Average **before** disinfection | **563** | **5.28** | **5.6** |
| Standard deviation | 0.44 | 0.38 | 0.39 |
| Average **after** disinfection | **2.75** | **2.58** | **3.02** |
| Standard deviation | 0·08 | 0·23 | 0.65 |
| **RF** | **2.88** | **2.70** | **2.58** |
| Standard deviation | 0.45 | 0.43 | 0.73 |
| **Number of test persons** | **5** | **5** | **5** |

The above data show that the combinations of isopropanol (75% by weight) with relipidizing agents (formulation B), according to the invention, have a better effect than 70% ethanol. In addition, it was surprising that the effect of a disinfection agent according to the invention with the combination of relipidizing agents (B) is even better than in the presence of glycerol as the sole relipidizing agent (formulation C).

### Claims

1. A composition **characterized in that** is consists of, for 100% by weight:

a) - At least 60% by weight and at most to 95% by weight of isopropanol, and

b) - At least 0.2% by weight and at most 4.0% by weight of relipidizing agents chosen from a mixture of cetyl alcohol and of stearyl alcohol or a mixture of cetyl alcohol, stearyl alcohol and myristyl alcohol;

c) - 0.05 to 2.0% by weight of at least one or several ingredients chosen from sorbitol, urea, dexpanthenol, 1-(2-ethyl hexyl) glycerol ether, a combination of sorbitol and dexpanthenol, or a combination of sorbitol, dexpanthenol and 1-(2-ethyl hexyl) glycerol ether, and

d) - water as the remainder.

2. A composition according to claim 1, **characterized in that** the component b) is a mixture of cetyl alcohol, stearyl alcohol and myristyl alcohol.

3. A composition according to claim 1, **characterized in that** the component c) is a combination of sorbitol and dexpanthenol, or a combination of sorbitol, dexpanthenol and 1-(2-ethyl hexyl) glycerol ether.

4. A composition according to any one of claims 1 to 3 for use in a method for treatment of the human or animal body by therapy.

5. A composition according to any one of claims 1 to 3 for use as a skin disinfectant in a method for treatment of the human or animal body by therapy.

6. A composition according to any one of claims 1 to 3 for use as a hygienic or surgical hand disinfectant.


**Patentansprüche**

1. Zusammensetzung, **dadurch gekennzeichnet, dass** sie auf 100 Gewichts-% besteht aus

a) - zumindest 60 Gewichts-% und höchstens bis 95 Gewichts-% Isopropanol, und

b) - zumindest 0,2 Gewichts-% und höchstens 4.0 Gewichts-% Rückfetter, ausgewählt aus einer Mischung von Cetylalkohol und von Stearylalkohol oder einer Mischung von Cetylalkohol, Stearylalkohol und Myristylalkohol;

c) - 0,05 bis 2,0 Gewichts-% von zumindest einem oder einigen Inhaltsstoffen, ausgewählt aus Sorbitol, Harnstoff, Dexpanthenol, 1-(2-Ethylhexyl)glycerinether, einer Kombination von Sorbitol und Dexpanthenol, oder einer Kombination von Sorbitol, Dexpanthenol und 1-(2-Ethylhexyl)glycerinether, und

d) - Wasser als den Rest.

2. Zusammensetzung gemäß Anspruch 1, **dadurch gekennzeichnet, dass** der Bestandteil b) eine Mischung von Cetylalkohol, Stearylalkohol und Myristylalkohol ist.

3. Zusammensetzung gemäß Anspruch 1, **dadurch gekennzeichnet, dass** der Bestandteil c) eine Kombination von Sorbitol und Dexpanthenol, oder eine Kombination von Sorbitol, Dexpanthenol und 1-(2-Ethylhexyl)glycerinether ist.

4. Zusammensetzung gemäß irgendeinem der Ansprüche 1 bis 3 zur Verwendung in einem Verfahren für die therapeutische Behandlung des menschlichen oder tierischen Körpers.

5. Zusammensetzung gemäß irgendeinem der Ansprüche 1 bis 3 zur Verwendung als ein Hautdesinfektionsmittel in einem Verfahren für die therapeutische Behandlung des menschlichen oder tierischen Körpers.

6. Zusammensetzung gemäß irgendeinem der Ansprüche 1 bis 3 zur Verwendung als ein hygienisches oder chirurgisches Hautdesinfektionsmittel.


**Revendications**

1. Composition **caractérisée en ce qu'**elle consiste en, pour 100 % en poids :

a) - au moins 60 % en poids et au plus 95 % en poids d'isopropanol, et

b) - au moins 0,2 % en poids et au plus 4,0 % en poids d'agents de relipidation choisis parmi un mélange d'alcool cétylique et d'alcool stéarylique ou d'un mélange d'alcool cétylique, d'alcool stéarylique et d'alcool myristylique ;

c) - de 0,05 à 2,0 % en poids d'au moins un ou plusieurs ingrédients sélectionnés parmi le sorbitol, l'urée, le dexpanthénol, l'éther de 1-(2-éthyl hexyl)glycérol, une combinaison de sorbitol et de dexpanthénol, ou une combinaison de sorbitol, de dexpanthénol et d'éther de 1-(2-éthyl hexyl) glycérol, et

d)- de l'eau en tant que complément.

2. Composition selon la revendication 1, **caractérisée** ce que le composant b) est un mélange d'alcool cétylique, d'alcool stéarylique et d'alcool myristylique.

3. Composition selon la revendication 1, **caractérisée en ce que** le composant c) est une combinaison de sorbitol et de dexpanthénol, ou une combinaison de sorbitol, de dexpanthénol et d'éther de 1-(2-éthyl hexyl)glycérol.

4. Composition selon l'une quelconque des revendications 1 à 3, pour son utilisation dans un procédé de traitement du corps humain ou animal par thérapie.

5. Composition selon l'une quelconque des revendications 1 à 3, pour son utilisation en tant que désinfectant pour la peau dans un procédé de traitement du corps humain ou animal par thérapie.

6. Composition selon l'une quelconque des revendications 1 à 3, pour son utilisation en tant que désinfectant hygiénique ou chirurgical pour les mains.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0547727 A1
- WO 9844792 A1
- EP 2135507 A1
- EP 1685854 A1
- JP 200035506 A
- EP 0547727 A

### Non-patent literature cited in the description

- Kramer & Assadian. Publ. Thieme Verlag, 2008
- **MAGULSKI et al.** *BMC-Infectious Diseases,* 2009, vol. 9, 107
- *International Conference on Prevention and Infection Control,* 2011